# EUROPÄISCHE PATENTSCHRIFT

(11) **EP 3 807 258 B1**
(45) Veröffentlichungstag und Bekanntmachung des Hinweises auf die Patenterteilung: **20.10.2021**
(21) Anmeldenummer: 19730718.4
(22) Anmeldetag: 05.06.2019
(51) Int. Cl.: C09B 69/10, C09B 69/00, C07D 311/92, C07D 311/94, C09K 9/02, G02B 5/23

(54) **PHOTOCHROME ANNELLIERTE NAPHTHOPYRAN-SYSTEME MIT SPEZIELLEN SUBSTITUENTEN ZUR REALISIERUNG SEHR SCHNELLER AUFHELLGESCHWINDIGKEITEN**
PHOTOCHROMIC ANNELATED NAPHTHOPYRANE SYSTEMS WITH SPECIAL SUBSTITUENTS, FOR ATTAINING RAPID LIGHTENING SPEEDS
SYSTÈMES DE NAPHTOPYRANNES CONDENSÉS PHOTOCHROMES POURVUS DE SUBSTITUANTS SPÉCIAUX POUR ATTEINDRE DES VITESSES D'ÉCLAIRCISSEMENT TRÈS RAPIDES

(30) Priorität: 14.06.2018 DE 102018004790
(43) Veröffentlichungstag der Anmeldung: 21.04.2021
(73) Patentinhaber: Rodenstock GmbH, 80687 München (DE)
(72) Erfinder: WEIGAND, Udo, 81247 München (DE); ZINNER, Herbert, 85296 Rohrbach (DE); ROHLFING, Yven, 81547 München (DE)
(74) Vertreter: Müller-Boré & Partner Patentanwälte PartG mbB
(86) Internationale Anmeldenummer: PCT/EP2019/064652
(87) Internationale Veröffentlichungsnummer: WO 2019/238495

(56) Entgegenhaltungen:
- EP-A1- 1 112 264
- EP-A1- 2 788 340
- EP-A1- 3 010 924

## Beschreibung

Die vorliegende Erfindung betrifft neue photochrome, annellierte Naphthopyran-Systeme mit speziellen Substituenten R₁, mit deren Hilfe sich sehr schnelle Aufhellgeschwindigkeiten - ohne Einbußen bei der Eindunklungstiefe nach Anregung - realisieren lassen, sowie deren Verwendung in Kunststoffen aller Art.

Seit langem sind verschiedene Farbstoffklassen bekannt, die bei Bestrahlung mit UVA-Licht, insbesondere Sonnenstrahlen, reversibel ihre Farbe wechseln. Dies geht darauf zurück, dass diese Farbstoffmoleküle durch Lichtenergie in einen angeregten Zustand ("offene Form") übergehen, den sie bei Unterbrechung der Energiezufuhr wieder verlassen und in ihren Ausgangszustand ("geschlossene Form") zurückkehren. Zu diesen photochromen Farbstoffen gehören verschiedene Systeme mit Pyran-Kernstrukturen, die im Stand der Technik mit unterschiedlichen Grundsystemen und Substituenten bereits beschrieben wurden.

Pyrane, speziell Naphthopyrane und von diesen abgeleitete größere Ringsysteme, sind derzeit die am meisten bearbeitete Klasse photochromer Verbindungen. Obwohl bereits im Jahr 1966 erstmals zum Patent angemeldet (US 3,567,605), konnten erst in den 90er Jahren Verbindungen entwickelt werden, die für den Einsatz in Brillengläsern geeignet erschienen. Eine geeignete Verbindungsklasse von Pyranen sind zum Beispiel die 2,2-Diaryl-2H-naphtho[1,2-b]pyrane oder die 3,3-Diaryl-3H-naphtho[2,1-b]pyrane, die in angeregter Form verschiedene Eindunklungsfarben von gelb bis rot aufweisen.

Von diesen Grundsystemen abgeleitet sind annellierte Naphthopyrane von großem Interesse, die aufgrund ihres größeren Ringsystems längerwellig absorbieren und violette und blaue Eindunklungsfarben ergeben. Dazu wird üblicherweise ein Benzolring mit einer zusätzlichen Verbrückung in ortho-Stellung eingesetzt - in den hier vorgestellten erfindungsgemäßen Verbindungen die Brücke mit den Substituenten R₆ und R₇ für Verbindungen der Formel (I) bzw. mit den Substituenten R₁₀, R₁₁ und R₁₂ für Verbindungen der Formel (II).

Liegt - wie bei den erfindungsgemäßen Verbindungen der Formel (I) - eine einatomige Verbrückung vor, so ergibt sich ein an das Naphthopyran annellierter Fünfring. Beispiele finden sich in EP 0 792 468 und EP 0 906 366 für ein Kohlenstoff-Brückenatom ("Indenonaphthopyrane") sowie in EP 0 946 536 für ein Sauerstoff-Brückenatom.

In EP 0 912 908, EP 2 457 915, EP 2 471 794, EP 2 684 886, EP 2 788 340 sowie EP 2 872 517 werden Verbindungen beschrieben, bei denen an die Indenonaphthopyran-Kernstruktur mindestens ein weiteres Ringsystem annelliert ist.

Liegt - wie bei den erfindungsgemäßen Verbindungen der Formel (II) - eine zweiatomige Verbrückung vor, so ergibt sich ein an das Naphthopyran annellierter Sechsring. Verbindungen mit einer Brücke aus zwei Kohlenstoff-Atomen sind in EP 1 119 560 beschrieben. Neuere Anmeldungen hierzu sind EP 2 829 537 sowie EP 3 010 924.

EP 1 097 156 beschreibt Verbindungen mit einer zweiatomigen Brücke aus einem Kohlenstoff- und einem Sauerstoff-Atom, ebenso wie EP 2 178 883 und EP 2 760 869.

In EP 1 112 264 sind u.a. (nicht weiter annellierte) Indenonaphthopyran-Systeme mit längeren Polyalkoxy-Substituenten beschrieben.

Der vorliegenden Erfindung liegt daher die Aufgabe zugrunde, photochrome Farbstoffe bereitzustellen, die sich durch sehr schnelle Aufhellgeschwindigkeiten - ohne Einbußen bei der Eindunklungstiefe nach Anregung -auszeichnen sollen.

Diese Aufgabe wird durch die in den Ansprüchen gekennzeichneten Gegenstände gelöst.

Insbesondere werden photochrome annellierte Naphthopyrane gemäß der allgemeinen Formel (I) (II) bereitgestellt: 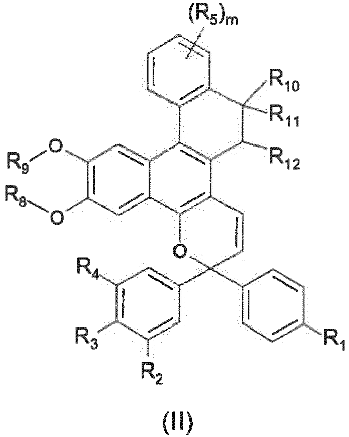 worin entweder nur der Rest R₁ oder R₁ und R₃ unabhängig voneinander folgende Gruppierung darstellt bzw. darstellen: 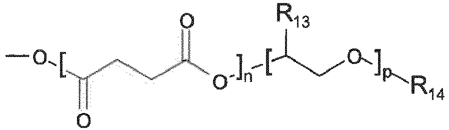
worin der Rest R₁₃ Wasserstoff oder einen Methyl-Rest darstellt und der Rest R₁₄ einen Substituenten, ausgewählt aus Wasserstoff, einem (C₁―C₆)-Alkyl-Rest, einem Acetyl-Rest, einem Benzoyl-Rest, einem Phenyl-Rest, einem Benzyl-Rest, einem Biphenyl-Rest, einem Naphthyl-Rest, einem tert-Butyl-dimethylsilyl-Rest oder einem tert-Butyl-diphenylsilyl-Rest, darstellt; n eine ganze Zahl von 0 bis 1 darstellt und p eine ganze Zahl von 3 bis 50 darstellt; oder falls R₁₃ einen Methyl-Rest darstellt, R₁₄ auch die Gruppierung -(CH₂-CH₂)_{q}-OR₁₅ darstellen kann, wobei der Rest R₁₅ aus Wasserstoff, einem (C₁― C₆)-Alkyl-Rest, einem Phenyl-Rest, einem Benzyl-Rest oder einem Biphenyl-Rest ausgewählt sein kann, wobei q eine ganze Zahl von 1 bis 20 darstellt; und
die Reste R₂, R₃ und R₄ jeweils unabhängig voneinander einen Substituenten darstellen, ausgewählt aus Wasserstoff, Brom, Chlor, Fluor, einem (C₁―C₆)-AlkylRest, einem (C₃―C₇)-Cycloalkyl-Rest, einem (C₁―C₆)-Thioalkyl-Rest, einem (C₁― C₁₈)-Alkoxy-Rest, einem Hydroxy-Rest, einem tert-Butyl-dimethylsilyloxy-Rest, einem tert-Butyl-diphenylsilyloxy-Rest, einem Trifluormethyl-Rest, einem Phenyl-Rest, einem 4-Methoxyphenyl-Rest, einem Phenoxy-Rest, einem 4-Methoxyphenoxy-Rest, einem Benzyl-Rest, einem 4-Methoxybenzyl-Rest, einem Benzyloxy-Rest, einem 4-Methoxybenzyloxy-Rest, einem Biphenyl-Rest, einem Biphenyloxy-Rest, einem Naphthyl-Rest, einem Naphthoxy-Rest, einem Mono-(Ci-C₆)-alkylamino-Rest, einem Di-(C₁-C₅)-alkylamino-Rest, einem Phenylamino-Rest, einem (C₁―C₆)-alkyl-phenylamino-Rest, einem Diphenylamino-Rest, einem (4-Methoxyphenyl)amino-Rest, einem ((C₁-C₆)-alkyl)-(4-methoxyphenyl)amino-Rest, einem Bis(4-methoxyphenyl)-amino-Rest, einem Piperidinyl-Rest, einem 3,5-Dimethylpiperidinyl-Rest, einem Indolinyl-Rest, einem Morpholinyl-Rest, einem 2,6-Dimethylmorpholinyl-Rest, einem Thiomorpholinyl-Rest, einem Azacycloheptyl-Rest, einem Phenothiazinyl-Rest, einem Phenoxazinyl-Rest, einem 1,2,3,4-Tetrahydrochinolinyl-Rest, einem 1,2,3,4-Tetrahydroisochinolinyl-Rest, einem Phenazinyl-Rest, einem Carbazolyl-Rest, einem 1,2,3,4-Tetrahydrocarbazolyl-Rest oder einem 10,11-Dihydro-dibenz[b,f]azepinyl-Rest;
oder die zwei benachbarten Reste R₂ und R₃ die Gruppierung -V-(CH₂)ᵣ-W-darstellen, wobei V und W unabhängig voneinander aus den Gruppierungen -O-, -S-, -N(C₁―C₆)-Alkyl-, -NC₆H₅-, -CH₂-,-C(CH₃)₂-, -C(C₂H₅)₂- oder -C(C₆H₅)₂-ausgewählt sind; r dabei eine ganze Zahl von 1 bis 3 darstellt; mit der Maßgabe, daß, wenn dieser Zahlenwert 2 oder 3 ist, an zwei benachbarten CH₂-Gruppen auch ein Benzol-Ring annelliert sein kann; V oder W zusammen mit der jeweils benachbarten CH₂-Gruppe auch einen annellierten Benzol-Ring darstellen kann;
oder, wie bereits vorstehend definiert, R₃ dieselbe Gruppierung wie R₁ darstellt;
die Reste R₅, R₈, R₉, R₁₀, R₁₁ und R₁₂ jeweils unabhängig voneinander einen Substituenten darstellen, ausgewählt aus Wasserstoff, einem (C₁―C₆)-Alkyl-Rest, einem (C₃―C₇)-Cycloalkyl-Rest, einem Phenyl-Rest, einem Benzyl-Rest, einem Biphenyl-Rest oder einem Naphthyl-Rest; wobei m eine ganze Zahl von 1 bis 3 darstellt;
oder zwei benachbarte Reste R₅ einen annellierten Benzol-Ring bilden, der un-, mono- oder disubstituiert sein kann, wobei die Substituenten ausgewählt sein können aus Wasserstoff, einem (C₁―C₆)-Alkyl-Rest, einem (C₁―C₆)-Alkoxy-Rest, einem Phenyl-Rest, einem Benzyl-Rest, einem Biphenyl-Rest oder einem Naphthyl-Rest;
oder zwei benachbarte Reste R₅ einen annellierten Benzofuran-Ring, einen annellierten Benzothiophen-Ring, einen annellierten 2H-Chromen-Ring, einen annellierten 3,3-Dimethylinden-Ring oder einen annellierten Dioxan-Ring bilden;
oder die Reste R₈ und R₉ zusammen die Gruppierung -(CH₂)ₛ- bilden, wobei s eine ganze Zahl von 1 bis 3 darstellt; mit der Maßgabe, dass, wenn dieser Zahlenwert 2 oder 3 ist, an zwei benachbarten CH₂-Gruppen auch ein Benzol-Ring annelliert sein kann.

Der vorliegenden Erfindung liegt die überraschende Feststellung zugrunde, dass durch die Einführung spezieller Substituenten R₁ in photochrome Farbstoff-Systeme aus dem Stand der Technik - aus der EP 2 788 340 für Verbindungen der Formel (I) bzw. aus der EP 3 010 924 für Verbindungen der Formel (II) - sich sehr schnelle Aufhellgeschwindigkeiten ohne Einbußen bei der Eindunklungstiefe nach Anregung realisieren lassen.

Bei den speziellen Substituenten R₁ handelt es sich um längere lineare Polyethylenoxy- bzw. Polypropylenoxy-Ketten, die optional über eine Succinyl-Esterbrücke kovalent an den photochromen Farbstoff gebunden sind. Diese Substituenten schirmen mit ihrer langen kettenförmigen Struktur offensichtlich die Farbstoff-Moleküle von der Kunststoff-Matrix so gut ab, dass dadurch eine von der Polarität und der Chemie der Kunststoff-Matrix unabhängige Umgebung geschaffen wird, welche die angeregte, farbige (offene) Form der Farbstoffe weniger stabilisiert. Dadurch kehren die photochromen Farbstoff-Moleküle nach Anregung schneller in ihren farblosen Grundzustand zurück, d.h. der Farbstoff hellt nach der Anregung schneller auf. Die langen kettenförmigen Strukturen der speziellen Substituenten R₁ wickeln sich um die Farbstoff-Moleküle und bauen sozusagen einen Schutzwall auf, der die Farbstoffe von der Kunststoff-Matrix abschirmt. Die Substituenten R₁ wirken deswegen besonders effizient, da sie nahe am photolabilen Zentrum des photochromen Farbstoffes sitzen und damit nahe am Ort des Bindungsbruches bei Anregung bzw. der Bindungs-Rückbildung bei der Aufhellung. Die Polarität der Ketten von R₁ ist dabei mitentscheidend - z.B. zeigen lange Paraffin-Ketten keinerlei Effekt.

Die erfindungsgemäßen Verbindungen zeichnen sich dadurch aus, dass durch die Einführung spezieller Substituenten R₁ in photochrome Farbstoff-Systeme aus dem Stand der Technik - aus EP 2 788 340 für Verbindungen der Formel (I) bzw. aus EP 3 010 924 für Verbindungen der Formel (II) - deutlich verbesserte Aufhell-Eigenschaften induziert werden können. Somit lassen sich die phototropen Eigenschaften dieser Farbstoff-Systeme deutlich verbessern, da die Eindunklungstiefe durch die Einführung der Substituenten R₁ nicht beeinflusst wird. Wie die zugrundeliegenden Systeme ohne die Substituenten R₁ zeichnen sich die erfindungsgemäßen Verbindungen durch ihre hervorragende UV-Härtungskompatibilität aus, d.h. sie überstehen - eingebracht z.B. in eine Acrylatmonomer-Matrix mit UV-Initiator - unbeschadet eine durch starkes UV-Licht ausgelöste radikalische Polymerisation der Matrix. Außerdem weisen sie bei sehr hoher Leistung eine sehr gute Lebensdauer aus. Sie sind in Kunststoffen aller Art einsetzbar.

Der Molekülstruktur der erfindungsgemäßen Verbindungen liegt eine Dihydronaphtho-Naphthopyran-Kernstruktur (mit den Substituenten R₅, R₈, R₉, R₁₀, R₁₁ und R₁₂) für Verbindungen der Formel (II) zugrunde, die jeweils die photolabile Pyran-Einheit (mit den Substituenten R₁, R₂, R₃ und R₄) enthält. Diese ist verantwortlich für den photochromen Charakter, da durch Lichtanregung die Bindung zwischen dem Sauerstoff der Pyran-Einheit und dem Kohlenstoffatom mit den beiden Aryl-Substituenten reversibel gebrochen wird, wodurch ein farbiges Merocyanin-System entsteht.

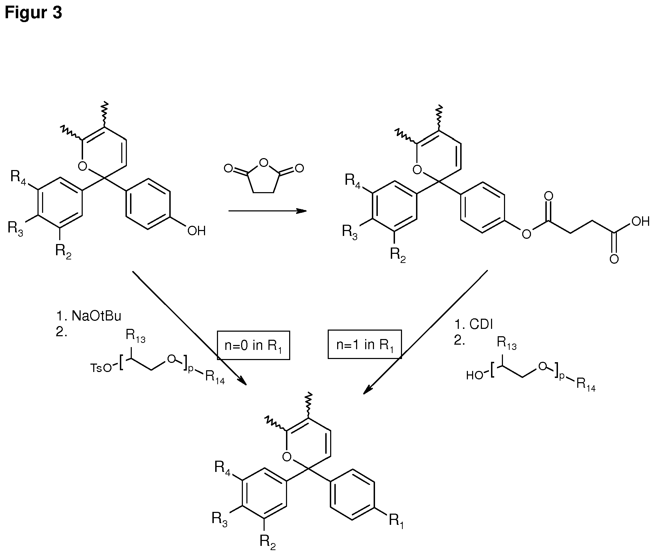

Die Synthese der erfindungsgemäßen Verbindungen gelingt gemäß Figur 3 aus den zugrundeliegenden Farbstoff-Systemen aus dem Stand der Technik mit einer Hydroxy-Gruppe an der Stelle von R₁.

Zur Messung der spektralen und photochromen Eigenschaften wurden jeweils äquimolare Mengen der erfindungsgemäßen Verbindungen sowie Verbindungen aus dem Stand der Technik in einer Acrylatmonomer-Matrix gelöst und nach Zusatz eines Polymerisations-Initiators mit Hilfe eines Temperaturprogrammes thermisch polymerisiert.

Das photochrome Eindunklungs- und Aufhellverhalten ("Kinetik-Diagramm") der so hergestellten Probekörper wurde anschließend gemäß DIN EN ISO 8980-3 ermittelt.

Figur 1 zeigt den Kinetik-Vergleich von Verbindungen mit der nachstehenden Formel (III) (nicht erfindungsgemäß, aber im Rahmen der vorliegenden Erfindung ebenso bereitgestellt) mit entsprechenden Verbindungen aus dem Stand der Technik aus EP 2 788 340.

Figur 2 zeigt den Kinetik-Vergleich erfindungsgemäßer Verbindungen der allgemeinen Formel (II), konkreter mit der nachstehenden Formel (IV), mit entsprechenden Verbindungen aus dem Stand der Technik aus EP 3 010 924.

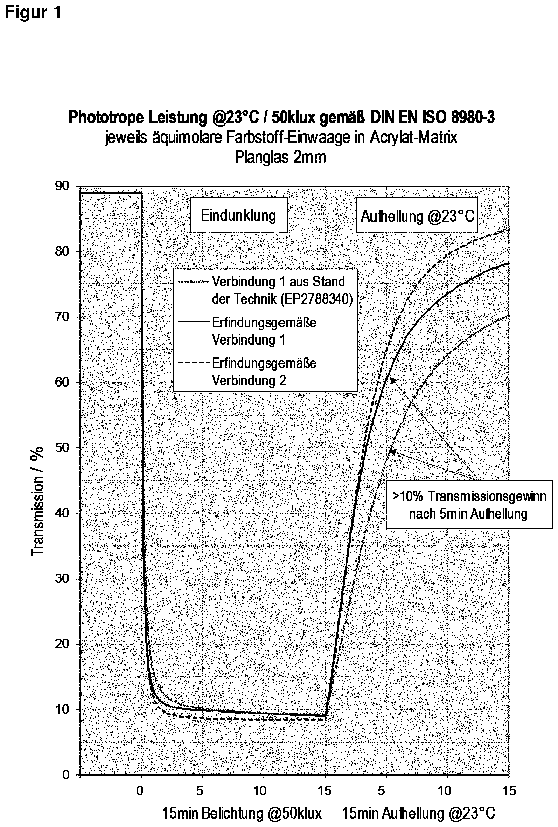

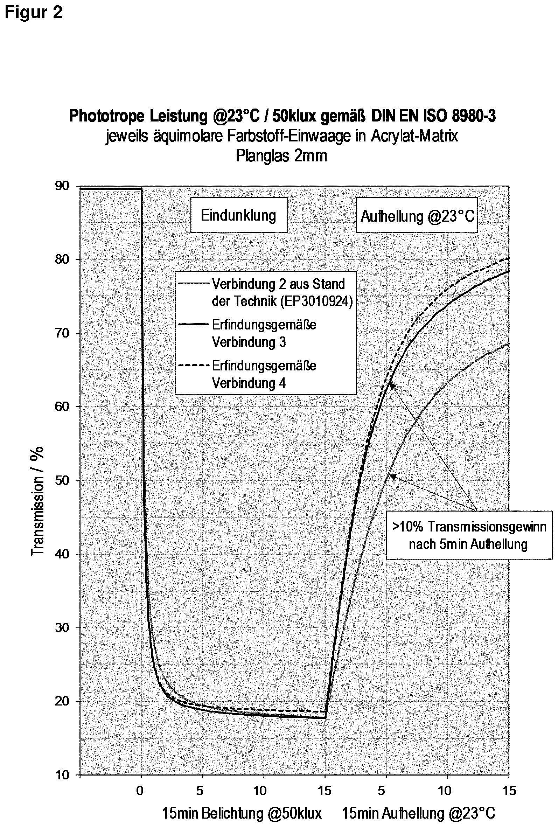

Die konkreten Molekülstrukturen der in der Figur 1 und 2 dargestellten Verbindungen sind in Tabelle 1 aufgeführt. Die Verbindungen 1 und 2 (nicht erfindungsgemäß) werden in der folgenden Tabelle 1 durch die Formel (III) beschrieben, die erfindungsgemäßen Verbindungen 3 und 4 durch die Formel (IV).

Die Figuren 1 und 2 zeigen anschaulich den deutlichen Effekt der erfindungsgemäßen speziellen Substituenten R₁ auf die Aufhellgeschwindigkeit, ohne Einbußen bei der Eindunklungstiefe.

**Tabelle 1: Molekülstrukturen der in Figur 1 und 2 vorgestellten Verbindungen**

| | | | | |
|---|---|---|---|---|
| 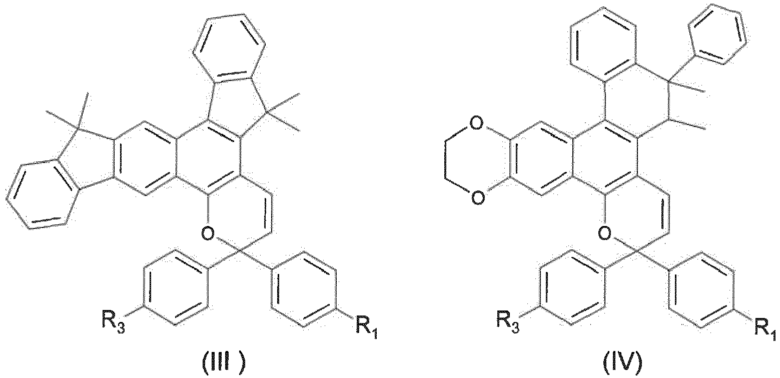 | | | | |

| | R₁ in Formel (III) | R₃ in ( III ) | R₁ in Formel ( IV) | R₃ in ( IV ) |
|---|---|---|---|---|
| Verbindung 1 aus Stand der Technik (EP 2 788 340) | OMe (=Methoxy) | OMe | | |
| Verbindung 1 | 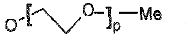 | OMe | | |
| | Me = Methyl; p≈16 (Gauss-Verteilung) | | | |
| Verbindung 2 | 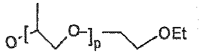 | OMe | | |
| | Et = Ethyl; p≈17 | | | |
| Verbindung 2 aus Stand der Technik (EP 3 010 924) | | | OMe | Me (=Methyl) |
| Erfindungsgemäße Verbindung 3 | | | 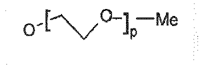 | Me |
| | | | Me = Methyl; p≈16 (Gauss-Verteilung) | |
| Erfindungsgemäße Verbindung 4 | | | 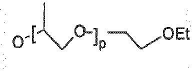 | Me |
| | | | Et = Ethyl; p≈17 | |

Die Verbindung 1 und die erfindungsgemäße Verbindung 3 weisen im Substituenten R₁ eine lineare Polyethylenoxy-Kette mit einer mittleren Kettenlänge von etwa 16 auf. Ausgangsmaterial für die Synthese ist im Handel erhältlicher Polyethylenglykolmonomethylether mit einem mittleren Molekulargewicht von 750. Dabei handelt es sich um eine Gauss-Verteilung verschiedener Kettenlängen mit einem Maximum bei etwa 16 Ethylenoxy-Einheiten.

Die Verbindung 2 und die erfindungsgemäße Verbindung 4 weisen im Substituenten R₁ eine lineare Polypropylenoxy-Kette mit einer mittleren Kettenlänge von etwa 17 auf. Ausgangsmaterial für die Synthese ist im Handel erhältliches Polypropylenglykol mit einem mittleren Molekulargewicht von 1000. Dabei handelt es sich ebenfalls um eine Gauss-Verteilung verschiedener Kettenlängen mit einem Maximum bei etwa 17 Propylenoxy-Einheiten. Die 2-Ethoxyethyl-Endgruppe wird mittels einer üblichen Williamson-Ethersynthese mit (2-Bromethyl)ethylether eingeführt.

Der Effekt der Beschleunigung der Aufhellgeschwindigkeit ist nicht nur für die vorstehend beschriebenen Kettenlängen beobachtbar; auch kürzere Ketten mit weniger als 10 Einheiten zeigen ebenfalls den Effekt.

Gemäß dem Syntheseschema in Figur 3 werden daraus dann die erfindungsgemäßen Verbindungen hersgestellt. In Figur 3 ist nur der Pyran-Ring mit den beiden Aryl-Substituenten dargestellt (n bezieht sich auf die Formel des Substituenten R₁ im Anspruch 1). Wenn n die Zahl 1 darstellt, werden die eben erwähnten längerkettigen Ausgangsverbindungen mittels einer Estersynthese kovalent an das photochrome Farbstoffmolekül angebunden. Die Carboxyl-Gruppe der Succinyl-Einheit wird mit CDI (Carbonyldiimidazol) aktiviert, was eine sehr milde Estersynthese erlaubt. Wenn n die Zahl 0 darstellt, muss die längerkettige Ausgangsverbindung zuerst als Tosylat (Ts) aktiviert werden. Danach gelingt die kovalente Anbindung an das photochrome Farbstoffmolekül mittels einer üblichen Williamson-Ethersynthese.

Beim Einsatz der erfindungsgemäßen Verbindungen in Kunststoffen aller Art können die photochromen Eigenschaften durch Zusatzstoffe weiter verbessert werden. Bevorzugt als Zusatzstoffe sind Verbindungen, die aus den gleichen strukturellen Einheiten wie Substituent R₁ aufgebaut sind und somit die chemische Umgebung der photolabilen Farbstoffmoleküle ähnlich gestalten können wie Substituent R₁, aber nicht diffusionsstabil fixiert sind. Bevorzugte Beispiele sind daher lineare Polyethylenglykole unterschiedlicher Kettenlänge, lineare Polypropylenglykole unterschiedlicher Kettenlänge, aus Ethylenoxy- und Propylenoxy-Einheiten alternierend aufgebaute Oligomere sowie Block-co-Polymere aus Ethylenoxy- und Propylenoxy-Einheiten. Als Endgruppen können neben Hydroxygruppen auch Alkylreste oder andere Gruppen eingesetzt werden.

Ein weiterer Gegenstand der vorliegenden Erfindung betrifft die Verwendung eines oder mehrerer der erfindungsgemäßen photochromen annellierten Naphthopyran-Systeme in Kunststoffen aller Art, insbesondere für ophthalmische Zwecke, in Linsen und Gläsern für Brillen aller Art, wie beispielsweise Korrektionsbrillen, Autofahrer-Brillen, Skibrillen, Sonnenbrillen, Motorradbrillen, für Visiere von Schutzhelmen und dergleichen und für Sonnenschutzzwecke in Fahrzeugen und im Baubereich, in Form von Fenstern, Schutzblenden, Abdeckungen, Dächern und dergleichen. Bei Verwendung in Kunststoffen aller Art können gemeinsam mit den erfindungsgemäßen Verbindungen Additive aller Art eingesetzt werden. Bevorzugt sind, wie vorstehend bereits ausgeführt, lineare Polyethylenglykole, lineare Polypropylenglykole, aus Ethylenoxy- und Propylenoxy-Einheiten alternierend aufgebaute Oligomere sowie Blockcopolymere aus Ethylenoxy- und Propylenoxy-Einheiten. Endgruppen der Oligomer-Additive können neben Hydroxygruppen auch Alkylgruppen oder andere Gruppen sein.

## Patentansprüche

1. Photochrome annellierte Naphthopyrane gemäß den Formeln ( I ) bzw. ( II ): 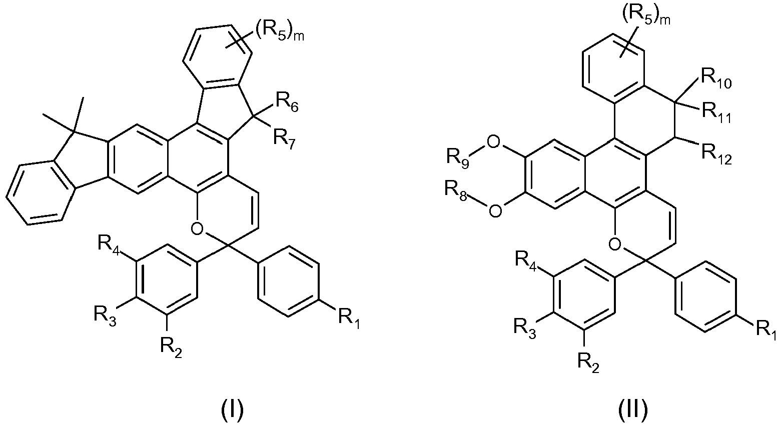 worin der Rest R₁ oder R₁ und R₃ unabhängig voneinander folgende Gruppierung darstellt: 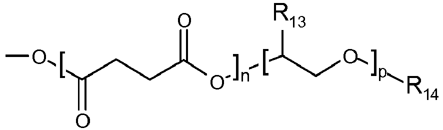
worin der Rest R₁₃ Wasserstoff oder einen Methyl-Rest darstellt und der Rest R₁₄ einen Substituenten, ausgewählt aus Wasserstoff, einem (C₁―C₆)-Alkyl-Rest, einem Acetyl-Rest, einem Benzoyl-Rest, einem Phenyl-Rest, einem Benzyl-Rest, einem Biphenyl-Rest, einem Naphthyl-Rest, einem tert-Butyl-dimethylsilyl-Rest oder einem tert-Butyl-diphenylsilyl-Rest, darstellt; n eine ganze Zahl von 0 bis 1 darstellt und p eine ganze Zahl von 3 bis 50 darstellt;
oder falls R₁₃ einen Methyl-Rest darstellt, R₁₄ auch die Gruppierung -(CH₂-CH₂)_{q}-OR₁₅ darstellen kann, wobei der Rest R₁₅ aus Wasserstoff, einem (C₁― C₆)-Alkyl-Rest, einem Phenyl-Rest, einem Benzyl-Rest oder einem Biphenyl-Rest ausgewählt sein kann, wobei q eine ganze Zahl von 1 bis 20 darstellt; und
die Reste R₂, R₃ und R₄ jeweils unabhängig voneinander einen Substituenten darstellen, ausgewählt aus Wasserstoff, Brom, Chlor, Fluor, einem (C₁―C₆)-AlkylRest, einem (C₃―C₇)-Cycloalkyl-Rest, einem (C₁―C₆)-Thioalkyl-Rest, einem (C₁― C₁₈)-Alkoxy-Rest, einem Hydroxy-Rest, einem tert-Butyl-dimethylsilyloxy-Rest, einem tert-Butyl-diphenylsilyloxy-Rest, einem Trifluormethyl-Rest, einem Phenyl-Rest, einem 4-Methoxyphenyl-Rest, einem Phenoxy-Rest, einem 4-Methoxyphenoxy-Rest, einem Benzyl-Rest, einem 4-Methoxybenzyl-Rest, einem Benzyloxy-Rest, einem 4-Methoxybenzyloxy-Rest, einem Biphenyl-Rest, einem Biphenyloxy-Rest, einem Naphthyl-Rest, einem Naphthoxy-Rest, einem Mono-(Ci-C₆)-alkylamino-Rest, einem Di-(C₁―C₆)-alkylamino-Rest, einem Phenylamino-Rest, einem (C₁―C₆)-alkyl-phenylamino-Rest, einem Diphenylamino-Rest, einem (4-Methoxyphenyl)amino-Rest, einem ((C₁―C₆)-alkyl)-(4-methoxyphenyl)amino-Rest, einem Bis(4-methoxyphenyl)-amino-Rest, einem Piperidinyl-Rest, einem 3,5-Dimethylpiperidinyl-Rest, einem Indolinyl-Rest, einem Morpholinyl-Rest, einem 2,6-Dimethylmorpholinyl-Rest, einem Thiomorpholinyl-Rest, einem Azacycloheptyl-Rest, einem Phenothiazinyl-Rest, einem Phenoxazinyl-Rest, einem 1,2,3,4-Tetrahydrochinolinyl-Rest, einem 1,2,3,4-Tetrahydroisochinolinyl-Rest, einem Phenazinyl-Rest, einem Carbazolyl-Rest, einem 1,2,3,4-Tetrahydrocarbazolyl-Rest oder einem 10,11-Dihydro-dibenz[b,f]azepinyl-Rest;
oder die zwei benachbarten Reste R₂ und R₃ die Gruppierung -V-(CH₂)ᵣ-W-darstellen, wobei V und W unabhängig voneinander aus den Gruppierungen -O-, -S-, -N(C₁―C₆)-Alkyl-, -NC₆H₅-, -CH2-,-C(CH3)2-, -C(C₂H₅)₂- oder -C(C₆H₅)₂-ausgewählt sind; r dabei eine ganze Zahl von 1 bis 3 darstellt; mit der Maßgabe, daß, wenn dieser Zahlenwert 2 oder 3 ist, an zwei benachbarten CH₂-Gruppen auch ein Benzol-Ring annelliert sein kann; V oder W zusammen mit der jeweils benachbarten CH₂-Gruppe auch einen annellierten Benzol-Ring darstellen kann;
oder R₃ dieselbe Gruppierung wie R₁ darstellt;
die Reste R₅, R₆, R7, R₉, R₉, R₁₀, R₁₁ und R₁₂ jeweils unabhängig voneinander einen Substituenten darstellen, ausgewählt aus Wasserstoff, einem (C₁―C₆)-Alkyl-Rest, einem (C₃―C₇)-Cycloalkyl-Rest, einem Phenyl-Rest, einem Benzyl-Rest, einem Biphenyl-Rest oder einem Naphthyl-Rest; wobei m eine ganze Zahl von 1 bis 3 darstellt;
oder zwei benachbarte Reste R₅ einen annellierten Benzol-Ring bilden, der un-, mono- oder disubstituiert sein kann, wobei die Substituenten ausgewählt sein können aus Wasserstoff, einem (C₁―C₆)-Alkyl-Rest, einem (C₁―C₆)-Alkoxy-Rest, einem Phenyl-Rest, einem Benzyl-Rest, einem Biphenyl-Rest oder einem Naphthyl-Rest;
oder zwei benachbarte Reste R₅ einen annellierten Benzofuran-Ring, einen annellierten Benzothiophen-Ring, einen annellierten 2H-Chromen-Ring, einen annellierten 3,3-Dimethylinden-Ring oder einen annellierten Dioxan-Ring bilden;
oder die Reste R₆ und R₇ zusammen mit dem an diese Reste gebundenen Kohlenstoffatom einen drei- bis achtgliedrigen carbo- oder heteromonocyclischen Ring bilden, an den ein bis zwei aromatische oder heteroaromatische Ringsysteme annelliert sein können, wobei das bzw. die Ringsysteme unabhängig voneinander ausgewählt sind aus Benzol, Naphthalin, Phenanthren, Pyridin, Chinolin, Furan, Thiophen, Pyrrol, Benzofuran, Benzothiophen, Indol und Carbazol;
oder die Reste R₈ und R₉ zusammen die Gruppierung -(CH₂)ₛ- bilden, wobei s eine ganze Zahl von 1 bis 3 darstellt; mit der Maßgabe, dass, wenn dieser Zahlenwert 2 oder 3 ist, an zwei benachbarten CH₂-Gruppen auch ein Benzol-Ring annelliert sein kann.

2. Photochrome annellierte Naphthopyrane gemäß Anspruch 1, worin der Rest R₁ die Gruppierung darstellt: 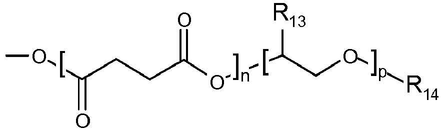
und der Rest R₃ einen Substituenten darstellt, ausgewählt aus Wasserstoff, Brom, Chlor, Fluor, einem (C₁―C₆)-Alkyl-Rest, einem (C₃―C₇)-Cycloalkyl-Rest, einem (C₁― C₆)-Thioalkyl-Rest, einem (C₁―C₁₈)-Alkoxy-Rest, einem Hydroxy-Rest, einem tert-Butyl-dimethylsilyloxy-Rest, einem tert-Butyl-diphenylsilyloxy-Rest, einem Trifluormethyl-Rest, einem Phenyl-Rest, einem 4-Methoxyphenyl-Rest, einem Phenoxy-Rest, einem 4-Methoxyphenoxy-Rest, einem Benzyl-Rest, einem 4-Methoxybenzyl-Rest, einem Benzyloxy-Rest, einem 4-Methoxybenzyloxy-Rest, einem Biphenyl-Rest, einem Biphenyloxy-Rest, einem Naphthyl-Rest, einem Naphthoxy-Rest, einem Mono-(C₁―C₆)-alkylamino-Rest, einem Di-(C₁―C₆)-alkylamino-Rest, einem Phenylamino-Rest, einem (C₁―C₆)-alkyl-phenylamino-Rest, einem Diphenylamino-Rest, einem (4-Methoxyphenyl)amino-Rest, einem ((C₁―C₆)-alkyl)-(4-methoxyphenyl)amino-Rest, einem Bis(4-methoxyphenyl)-amino-Rest, einem Piperidinyl-Rest, einem 3,5-Dimethylpiperidinyl-Rest, einem Indolinyl-Rest, einem Morpholinyl-Rest, einem 2,6-Dimethylmorpholinyl-Rest, einem Thiomorpholinyl-Rest, einem Azacycloheptyl-Rest, einem Phenothiazinyl-Rest, einem Phenoxazinyl-Rest, einem 1,2,3,4-Tetrahydrochinolinyl-Rest, einem 1,2,3,4-Tetrahydroisochinolinyl-Rest, einem Phenazinyl-Rest, einem Carbazolyl-Rest, einem 1,2,3,4-Tetrahydrocarbazolyl-Rest oder einem 10,11-Dihydro-dibenz[b,f]azepinyl-Rest; und
worin die anderen Reste wie vorstehend definiert sind.

3. Photochrome annellierte Naphthopyrane gemäß Anspruch 1 oder 2, worin die Reste R₅, R₆, R₇, R₉, R₉, R₁₀, R₁₁ und R₁₂ jeweils unabhängig voneinander einen Substituenten darstellen, ausgewählt aus Wasserstoff, einem (C₁―C₆)-Alkyl-Rest, einem (C₃―C₇)-Cycloalkyl-Rest, einem Phenyl-Rest, einem Benzyl-Rest, einem Biphenyl-Rest oder einem Naphthyl-Rest; wobei m eine ganze Zahl von 1 bis 3 darstellt.

4. Photochrome annellierte Naphthopyrane gemäß einem der Ansprüche 1 bis 3, worin der Rest R₁ folgende Gruppierung darstellt: 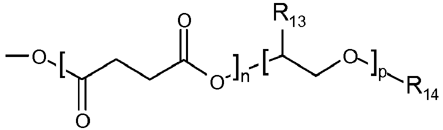
worin der Rest R₁₃ Wasserstoff oder einen Methyl-Rest darstellt und der Rest R₁₄ einen Substituenten, ausgewählt aus Wasserstoff, einem (C₁―C₆)-Alkyl-Rest, einem Acetyl-Rest, einem Benzoyl-Rest, einem Phenyl-Rest, einem Benzyl-Rest, einem Biphenyl-Rest, einem Naphthyl-Rest, einem tert-Butyl-dimethylsilyl-Rest oder einem tert-Butyl-diphenylsilyl-Rest, darstellt; n eine ganze Zahl von 0 bis 1 darstellt und p eine ganze Zahl von 3 bis 50 darstellt;
oder falls R₁₃ einen Methyl-Rest darstellt, R₁₄ auch die Gruppierung -(CH₂-CH₂)_{q}-OR₁₅ darstellen kann, wobei der Rest R₁₅ aus Wasserstoff, einem (C₁― C₆)-Alkyl-Rest, einem Phenyl-Rest, einem Benzyl-Rest oder einem Biphenyl-Rest ausgewählt sein kann, wobei q eine ganze Zahl von 1 bis 20 darstellt; und
der Rest R₃ einen Substituenten darstellt, ausgewählt aus Wasserstoff, Brom, Chlor, Fluor, einem (C₁―C₆)-Alkyl-Rest, einem (C₃―C₇)-Cycloalkyl-Rest, einem (C₁―C₆)-Thioalkyl-Rest, einem (C₁―C₁₈)-Alkoxy-Rest, einem Hydroxy-Rest, einem tert-Butyl-dimethylsilyloxy-Rest, einem tert-Butyl-diphenylsilyloxy-Rest, einem Trifluormethyl-Rest, einem Phenyl-Rest, einem 4-Methoxyphenyl-Rest, einem Phenoxy-Rest, einem 4-Methoxyphenoxy-Rest, einem Benzyl-Rest, einem 4-Methoxybenzyl-Rest, einem Benzyloxy-Rest, einem 4-Methoxybenzyloxy-Rest, einem Biphenyl-Rest, einem Biphenyloxy-Rest, einem Naphthyl-Rest, einem Naphthoxy-Rest, einem Mono-(C₁―C₆)-alkylamino-Rest, einem Di-(C₁―C₆)-alkylamino-Rest, einem Phenylamino-Rest, einem (C₁―C₆)-alkyl-phenylamino-Rest, einem Diphenylamino-Rest, einem (4-Methoxyphenyl)amino-Rest, einem ((C₁―C₆)-alkyl)-(4-methoxyphenyl)amino-Rest, einem Bis(4-methoxyphenyl)-amino-Rest, einem Piperidinyl-Rest, einem 3,5-Dimethylpiperidinyl-Rest, einem Indolinyl-Rest, einem Morpholinyl-Rest, einem 2,6-Dimethylmorpholinyl-Rest, einem Thiomorpholinyl-Rest, einem Azacycloheptyl-Rest, einem Phenothiazinyl-Rest, einem Phenoxazinyl-Rest, einem 1,2,3,4-Tetrahydrochinolinyl-Rest, einem 1,2,3,4-Tetrahydroisochinolinyl-Rest, einem Phenazinyl-Rest, einem Carbazolyl-Rest, einem 1,2,3,4-Tetrahydrocarbazolyl-Rest oder einem 10,11-Dihydro-dibenz[b,f]azepinyl-Rest, vorzugsweise ausgewählt aus Wasserstoff, einem (C₁―C₆)-Alkyl-Rest, einem (C₃―C₇)-Cycloalkyl-Rest, einem (C₁―C₆)-Thioalkyl-Rest, einem (C₁―C₆)-Alkoxy-Rest, einem Hydroxy-Rest, einem tert-Butyl-dimethylsilyloxy-Rest, einem tert-Butyl-diphenylsilyloxy-Rest, einem Trifluormethyl-Rest, einem Phenyl-Rest, einem 4-Methoxyphenyl-Rest, einem Phenoxy-Rest, einem 4-Methoxyphenoxy-Rest, einem Benzyl-Rest, einem 4-Methoxybenzyl-Rest, einem Benzyloxy-Rest oder einem 4-Methoxybenzyloxy-Rest.

5. Verwendung eines oder mehrerer der photochromen annellierten Naphthopyrane gemäß einem der vorhergehenden Ansprüche 1-4 in Kunststoffen aller Art, insbesondere für ophthalmische Zwecke, in Linsen und Gläsern für Brillen aller Art, vorzugsweise Korrektionsbrillen, Autofahrer-Brillen, Skibrillen, Sonnenbrillen, Motorradbrillen, für Visiere von Schutzhelmen und dergleichen und für Sonnenschutzzwecke in Fahrzeugen und im Baubereich, in Form von Fenstern, Schutzblenden, Abdeckungen, Dächern.

## Claims

1. Photochromic fused naphthopyrans according to formulae (I) and (II) respectively: 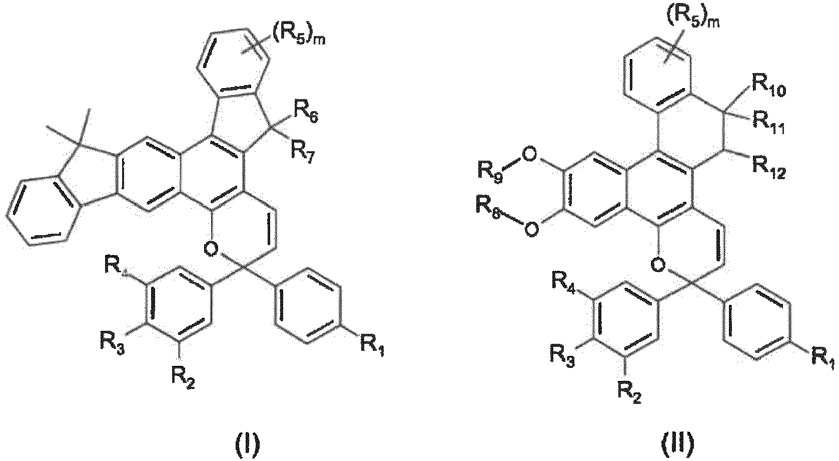 wherein R₁ or R₁ and R₃ independently represent the following grouping: 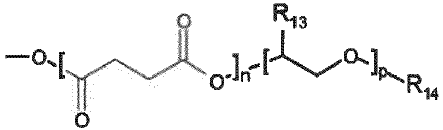
wherein R₁₃ represents hydrogen or a methyl radical and R₁₄ represents a substituent selected from hydrogen, a (C₁-C₆)-alkyl radical, an acetyl radical, a benzoyl radical, a phenyl radical, a benzyl radical, a biphenyl radical, a naphthyl radical, a tert-butyl-dimethylsilyl radical or a tert-butyl-diphenylsilyl radical; n represents an integer of 0 to 1; and p represents an integer of 3 to 50;
or if R₁₃ represents a methyl radical, R₁₄ also represents the grouping -(CH₂-CH₂)_{q}-OR₁₅ wherein R₁₅ may be selected from hydrogen, a (C₁-C₆)-alkyl radical, a phenyl radical, a benzyl radical or a biphenyl radical, wherein q represents an integer from 1 to 20; and the radicals R₂, R₃ and R₄ each independently represent a substituent selected from hydrogen, bromine, chlorine, fluorine, a (C₁-C₆)-alkyl radical, a (C₃-C₇)-cycloalkyl radical, a (C₁-C₆)-thioalkyl radical, a (C₁-C₁₈)-alkoxy radical, a hydroxy radical, a tert-butyl-dimethylsilyloxy radical, a tert-butyl-diphenylsilyloxy radical a trifluoromethyl radical, a phenyl radical, a 4-methoxyphenyl radical, a phenoxy radical, a 4-methoxyphenoxy radical, a benzyl radical, a 4-methoxybenzyl radical, a benzyloxy radical, a 4-methoxybenzyloxy radical, a biphenyl radical, a biphenyloxy radical, a naphthyl radical, a naphthoxy radical, a mono-(C₁-C₆)alkylamino radical, a di-( C₁-C₆)alkylamino radical, a di-(C₁-C₆)alkylamino radical, a di-(C₁-C₆)alkylamino radical, a di-( C₁-C₆)alkylamino radical, a di-(C₁-C₆)alkylamino radical, a di-(C₁-C₆)alkylamino radical, a di-(C₁-C₆)alkylamino radical, a di-(C₁-C₆)amino radical a di-(C₁-C₆)-alkylamino radical, a phenylamino radical, a (C₁-C₆)-alkyl-phenylamino radical, a diphenylamino radical, a (4- methoxyphenyl)amino radical a ((C₁-C₆)alkyl)-(4-methoxyphenyl)amino radical, a bis(4-methoxyphenyl)amino radical, a piperidinyl radical, a 3,5-dimethylpiperidinyl radical, an indolinyl radical, a morpholinyl radical, a 2,6-dimethylmorpholinyl radical, a thiomorpholinyl radical, an azacycloheptyl radical, a phenothiazinyl radical, a phenoxazinyl radical, a 1,2,3,4-tetrahydroquinolinyl radical a 1,2,3,4-tetrahydroisoquinolinyl radical, a phenazinyl radical, a carbazolyl radical, a 1,2,3,4-tetrahydrocarbazolyl radical or a 10,11 -dihydro-dibenz[b,f]azepinyl radical;
or the two adjacent radicals R₂ and R₃ represent the grouping -V-(CH₂)ᵣ-W-, where V and W are selected independently of one another from the groupings -O-, -S-, -N(C₁-C₆)-alkyl-, - NC₆H₅-, -CH₂ -, -C(CH₃)₂-, -C(C₂H₅)₂- or -C(C₆H₅)₂-; r represents an integer from 1 to 3; with the proviso that when this numerical value is 2 or 3, a benzene ring can also be fused to two adjacent CH₂ groups; V or W together with the respective adjacent CH₂ group can also represent a fused benzene ring;
or R₃ represents the same grouping as R₁;
R₅, R₆, R₇, R₈, R₉, R₁₀, R₁₁ and R₁₂ each independently represents a substituent selected from hydrogen, a (C₁-C₆)-alkyl group, a (C₃-C₇)-cycloalkyl group, a phenyl group, a benzyl group, a biphenyl group or a naphthyl group; wherein m represents an integer of from 1 to 3;
or two adjacent radicals R₅ form a fused benzene ring which may be unsubstituted, mono- or disubstituted, wherein the substituents may be selected from hydrogen, a (C₁-C₆)-alkyl radical, a (C₁-C₆)alkoxy radical, a phenyl radical, a benzyl radical, a biphenyl radical or a naphthyl radical;
or two adjacent radicals R₅ form a fused benzofuran ring, a fused benzothiophene ring, a fused 2H-chromene ring, a fused 3,3-dimethylindene ring or a fused dioxane ring;
or the radicals R₆ and R₇ together with the carbon atom attached to these radicals form a three- to eight-membered carbo- or heteromonocyclic ring to which one to two aromatic or heteroaromatic ring systems may be fused, the ring system(s) being independently selected from benzene, naphthalene, phenanthrene, pyridine, quinoline, furan, thiophene, pyrrole, benzofuran, benzothiophene, indole and carbazole;
or the radicals R₈ and R₉ together form the grouping -(CH₂)ₛ-, where s represents an integer from 1 to 3; with the proviso that if this numerical value is 2 or 3, a benzene ring may also be fused to two adjacent CH₂ groups.

2. Photochromic fused naphthopyrans according to claim 1, wherein the radical R₁ represents the grouping: 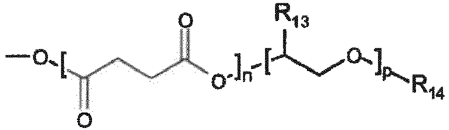
and R₃ is a substituent selected from hydrogen, bromine, chlorine, fluorine, (C₁-C₆)alkyl, (C₃-C₇)cycloalkyl, (C₁-C₆)thioalkyl, (C₁-C₁₈)alkoxy, hydroxy, tert-butyl-dimethylsilyloxy, tert-butyl-diphenylsilyloxy, trifluoromethyl, a phenyl radical, a 4-methoxyphenyl radical, a phenoxy radical, a 4-methoxyphenoxy radical, a benzyl radical, a 4-methoxybenzyl radical, a benzyloxy radical, a 4-methoxybenzyloxy radical, a biphenyl radical, a biphenyloxy radical, a naphthyl radical, a naphthoxy radical, a mono-( C₁-C₆)alkylamino radical, a di-( C₁-C₆)alkylamino radical, a phenylamino radical, a (C₁-C₆)-alkyl-phenylamino radical, a diphenylamino radical, a (4-methoxyphenyl)amino radical, a ((C₁-C₆)-alkyl)-(4-methoxyphenyl)amino radical a bis(4-methoxyphenyl)amino radical, a piperidinyl radical, a 3,5-dimethylpiperidinyl radical, an indolinyl radical, a morpholinyl radical, a 2,6-dimethylmorpholinyl radical a thiomorpholinyl radical, an azacycloheptyl radical, a phenothiazinyl radical, a phenoxazinyl radical, a 1 ,2,3,4-tetrahydroquinolinyl radical, a 1,2,3,4-tetrahydroisoquinolinyl radical, a phenazinyl radical, a carbazolyl radical, a 1,2,3,4-tetrahydrocarbazolyl radical or a 10,11 -dihydro-dibenz[b,f]azepinyl radical; and
wherein the other residues are as defined above.

3. Photochromic fused naphthopyrans according to claim 1 or 2, wherein R₅, R₆, R₇, R₉, R₉, R₁₀, R₁₁ and R₁₂ are each independently a substituent selected from hydrogen, a (C₁-C₆)alkyl radical, a (C₃-C₇)cycloalkyl radical, a phenyl radical, a benzyl radical, a biphenyl radical or a naphthyl radical; m being an integer from 1 to 3.

4. Photochromic fused naphthopyrans according to any one of claims 1 to 3, wherein R₁ represents the following grouping: 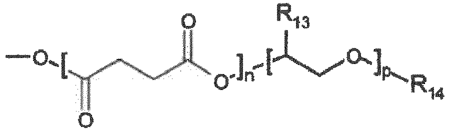
wherein R₁₃ represents hydrogen or a methyl radical and R₁₄ represents a substituent selected from hydrogen, a (C₁-C₆)alkyl radical, an acetyl radical, a benzoyl radical, a phenyl radical, a benzyl radical, a biphenyl radical, a naphthyl radical, a tert-butyl-dimethylsilyl radical or a tert-butyl-diphenylsilyl radical; n represents an integer of 0 to 1; and p represents an integer of 3 to 50;
or if R₁₃ represents a methyl radical, R₁₄ also represents the grouping -(CH₂-CH₂)_{q}-OR₁₅ wherein R₁₅ may be selected from hydrogen, a (C₁-C₆)alkyl radical, a phenyl radical, a benzyl radical or a biphenyl radical, wherein q represents an integer from 1 to 20; and
R₃ is a substituent selected from hydrogen, bromine, chlorine, fluorine, (C₁-C₆)alkyl, (C₃-C₇)cycloalkyl, (C₁-C₆)thioalkyl, (C1-C8)alkoxy, hydroxy, tert-butyl-dimethylsilyloxy, tert-butyl-diphenylsilyloxy, trifluoromethyl, phenyl, phenylsilyloxy, tert-butyl-diphenylsilyloxy, tert-butylsilyloxy, tert-butylsilyloxy a tert-butyl-dimethylsilyloxy radical, a tert-butyl-diphenylsilyloxy radical, a trifluoromethyl radical, a phenyl radical, a 4-methoxyphenyl radical, a phenoxy radical, a 4-methoxyphenoxy radical, a benzyl radical, a 4-methoxybenzyl radical, a benzyloxy radical, a 4-methoxybenzyloxy radical, a biphenyl radical, a biphenyloxy radical, a naphthyl radical, a naphthoxy radical, a mono-( C₁-C₆)-alkylamino radical, a di-( C₁-C₆)-alkylamino radical a phenylamino radical, a (C₁-C₆)-alkyl-phenylamino radical, a diphenylamino radical, a (4-methoxyphenyl)amino radical, a ((C₁-C₆)-alkyl)-(4-methoxyphenyl)amino radical, a bis(4-methoxyphenyl)amino radical, a piperidinyl radical a 3,5-dimethylpiperidinyl radical, an indolinyl radical, a morpholinyl radical, a 2,6-dimethylmorpholinyl radical, a thiomorpholinyl radical, an azacycloheptyl radical, a phenothiazinyl radical, a phenoxazinyl radical, a 1 ,2,3,4-tetrahydroquinolinyl radical, a 1 ,2,3,4-tetrahydroisoquinolinyl radical, a phenazinyl radical, a carbazolyl radical, a 1 ,2,3,4-tetrahydrocarbazolyl radical or a 10,1 1 -dihydro-dibenz[b,f]azepinyl radical, preferably selected from hydrogen, a (C₁-C₆)-alkyl radical, a (C₃-C₇)-cycloalkyl radical, a (C₁-C₆)-thioalkyl radical, a (C₁-C₆)-alkoxy radical, a hydroxy radical, a tert-butyl-dimethylsilyloxy radical, a tert-butyl-diphenylsilyloxy radical a trifluoromethyl radical, a phenyl radical, a 4-methoxyphenyl radical, a phenoxy radical, a 4-methoxyphenoxy radical, a benzyl radical, a 4-methoxybenzyl radical, a benzyloxy radical or a 4-methoxybenzyloxy radical.

5. Use of one or more of the photochromic annelated naphthopyrans according to one of the preceding claims 1 - 4 in plastics of all kinds, in particular for ophthalmic purposes, in lenses and glasses for spectacles of all kinds, preferably prescription spectacles, motorists' spectacles, ski goggles, sunglasses, motorbike goggles, for visors of protective helmets and the like and for sun protection purposes in vehicles and in the building sector, in the form of windows, protective screens, covers, roofs.

## Revendications

1. Naphtopyrannes fusionnés photochromiques selon les formules (I) et (II) respectivement : 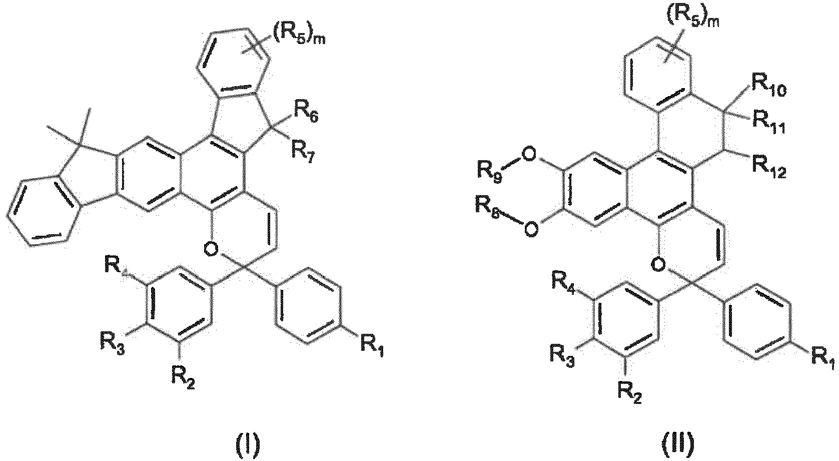 dans laquelle R₁ ou R₁ et R₃ représentent indépendamment le groupement suivant : 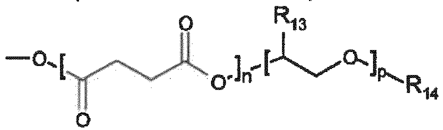
dans laquelle R₁₃ représente l'hydrogène ou un radical méthyle et R₁₄ représente un substituant choisi parmi l'hydrogène, un radical alkyle en C₁-C₆, un radical acétyle, un radical benzoyle, un radical phényle, un radical benzyle, un radical biphényle, un radical naphtyle, un radical tert-butyl-diméthylsilyle ou un radical tert-butyl-diphénylsilyle ; n représente un nombre entier de 0 à 1 ; et p représente un nombre entier de 3 à 50 ;
ou si R₁₃ représente un radical méthyle, R₁₄ représente également le groupement (-(CH₂-CH₂)_{q}-OR₁₅ où R₁₅ peut être choisi parmi l'hydrogène, un radical alkyle en C₁-C₆, un radical phényle, un radical benzyle ou un radical biphényle, où q représente un nombre entier de 1 à 20 ; et les radicaux R₂, R₃ et R₄ représentent chacun indépendamment un substituant choisi parmi l'hydrogène, le brome, le chlore, le fluor, un radical alkyle en C₁-C₆, un radical cycloalkyle en C₃-C₇, un radical thioalkyle en C₁-C₆, un radical alcoxy en C₁-C₁₈, un radical hydroxy, un radical tert-butyl-diméthylsilyloxy, un radical tert-butyl-diphénylsilyloxy un radical trifluorométhyle, un radical phényle, un radical 4-méthoxyphényle, un radical phénoxy, un radical 4-méthoxyphénoxy, un radical benzyle, un radical 4-méthoxybenzyle, un radical benzyloxy, un radical 4-méthoxybenzyloxy, un radical biphényle, un radical biphényloxy, un radical naphtyle, un radical naphtoxy, un radical monoalkyl(C₁-C₆)amino, un radical dialkyl(C₁-C₆)amino, un radical dialkyl(C₁-C₆)amino, un radical dialkyl(C₁-C₆)amino, un radical dialkyl(C₁-C₆)amino, un radical dialkyl(C₁-C₆)amino, un radical dialkyl(C₁-C₆)amino, un radical dialkyl(C₁-C₆)amino, un radical dialkyl(C₁-C₆)amino, un radical dialkyl(C₁-C₆)amino, un radical dialkyl(C₁-C₆)amino,un radical dialkyl(C₁-C₆)amino, un radical dialkyl(C₁-C₆6)amino, un radical dialkyl(C₁-C₆)amino,un radical dialkyl(C₁-C₆)amino,un radical dialkyl(C₁-C₆)amino,un radical dialkyl(C₁-C₆)amino un radical di-( C₁-C₆)-alkylamino, un radical phénylamino, un radical (C₁-C₆)-alkyl-phénylamino, un radical diphénylamino, un radical (4- méthoxyphényl)amino un radical ((alkyle en C₁-C₆)-(4-méthoxyphényl)amino, un radical bis(4-méthoxyphényl)amino, un radical pipéridinyle, un radical 3,5-diméthylpipéridinyle, un radical indolinyle, un radical morpholinyle, un radical 2,un radical 6-diméthylmorpholinyle, un radical thiomorpholinyle, un radical azacycloheptyle, un radical phénothiazinyle, un radical phénoxazinyle, un radical 1 , 2,3,4-tétrahydroquinolinyle un radical 1 ,2,3,4-tétrahydroisoquinolinyle, un radical phénazinyle, un radical carbazolyle, un radical 1 ,2,3,4-tétrahydrocarbazolyle ou un radical 10,1 1 -dihydro-dibenz[b,f]azépinyle ;
ou les deux radicaux adjacents R₂ et R₃ représentent le groupement -V-(CH₂)ᵣ-W-, où V et W sont choisis indépendamment l'un de l'autre parmi les groupements -O-, -S-, -N(C₁-C₆)-alkyle-, -NC₆H₅-, -CH₂-, -C(CH₃)₂-, -C(C₂H₅)₂- ou -C(C₆H₅)₂- ; r représente un nombre entier de 1 à 3 ; à condition que, lorsque cette valeur numérique est égale à 2 ou 3, un noyau benzénique puisse également être fusionné à deux groupes CH₂ adjacents ; V ou W, conjointement avec le groupe CH₂ adjacent respectif, peut également représenter un noyau benzénique fusionné ;
ou R₃ représente le même groupement que R₁ ;
R₅, R₆, R₇, R₈, R₉, R₁₀, R₁₁ et R₁₂ représentent chacun indépendamment un substituant choisi parmi l'hydrogène, un groupe alkyle en C₁-C₆, un groupe cycloalkyle en C₃-C₇, un groupe phényle, un groupe benzyle, un groupe biphényle ou un groupe naphtyle ; où m représente un entier de 1 à 3 ;
ou deux radicaux adjacents R₅ forment un cycle benzénique fusionné qui peut être non substitué, mono- ou disubstitué, où les substituants peuvent être choisis parmi l'hydrogène, un radical alkyle en C₁-C₆, un radical alcoxy en C₁-C₆, un radical phényle, un radical benzyle, un radical biphényle ou un radical naphtyle ;
ou deux radicaux adjacents R₅ forment un cycle benzofurane condensé, un cycle benzothiophène condensé, un cycle 2H-chromène condensé, un cycle 3,3-diméthylindène condensé ou un cycle dioxane condensé ;
ou bien les radicaux R₆ et R₇ forment, conjointement avec l'atome de carbone lié à ces radicaux, un noyau carbo- ou hétéromonocyclique de trois à huit chaînons auquel peuvent être condensés un ou deux systèmes cycliques aromatiques ou hétéroaromatiques, le ou les systèmes cycliques étant choisis indépendamment parmi le benzène, le naphtalène, le phénanthrène, la pyridine, la quinoléine, le furane, le thiophène, le pyrrole, le benzofurane, le benzothiophène, l'indole et le carbazole ;
ou bien les radicaux R₈ et R₉ forment ensemble le groupement -(CH₂)ₛ-, où s représente un nombre entier de 1 à 3 ; à condition que si cette valeur numérique est de 2 ou 3, un noyau benzénique puisse également être fusionné à deux groupes CH₂ adjacents.

2. Naphtopyrannes fusionnés photochromiques selon la revendication 1, dans lesquels le radical R₁ représente le groupement : 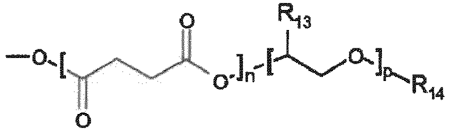
et R₃ est un substituant choisi parmi l'hydrogène, le brome, le chlore, le fluor, les groupes alkyle en C₁-C₆, cycloalkyle en C₃-C₇, thioalkyle en C₁-C₆, alcoxy en C₁-C₁₈, hydroxy, tert-butyl-diméthylsilyloxy, tert-butyl-diphénylsilyloxy, trifluorométhyle, un radical phényle, un radical 4-méthoxyphényle, un radical phénoxy, un radical 4-méthoxyphénoxy, un radical benzyle, un radical 4-méthoxybenzyle, un radical benzyloxy, un radical 4-méthoxybenzyloxy, un radical biphényle, un radical biphényloxy, un radical naphtyle, un radical naphtoxy, un radical mono-( C₁-C₆)alkylamino, un radical di-( C₁-C₆)alkylamino, un radical phénylamino, un radical (C₁-C₆)-alkyl-phénylamino, un radical diphénylamino, un radical (4-méthoxyphényl)amino, un radical ((C₁-C₆)-alkyl)-(4-méthoxyphényl)amino un radical bis(4-méthoxyphényl)amino, un radical pipéridinyle, un radical 3,5-diméthylpipéridinyle, un radical indolinyle, un radical morpholinyle, un radical 2,6-diméthylmorpholinyle un radical thiomorpholinyle, un radical azacycloheptyle, un radical phénothiazinyle, un radical phénoxazinyle, un radical 1 ,2,3,4-tétrahydroquinolinyle, un radical 1,2,3,4-tétrahydroisoquinolinyle, un radical phénazinyle, un radical carbazolyle, un radical 1,2,3,4-tétrahydrocarbazolyle ou un radical 10,11 -dihydro-dibenz[b,f]azépinyle ; et
où les autres résidus sont tels que définis ci-dessus.

3. Naphtopyrannes fusionnés photochromiques selon la revendication 1 ou 2, dans lesquels R₅, R₆, R₇, R₈, R₉, R₁₀, R₁₁ et R₁₂ sont chacun indépendamment un substituant choisi parmi l'hydrogène, un radical alkyle en C₁-C₆, un radical cycloalkyle en C₃-C₇, un radical phényle, un radical benzyle, un radical biphényle ou un radical naphtyle ; m étant un entier de 1 à 3.

4. Naphtopyrannes fusionnés photochromiques selon l'une quelconque des revendications 1 à 3, dans lesquels R₁ représente le groupement suivant : 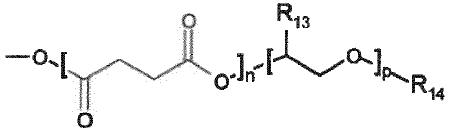
dans laquelle R₁₃ représente l'hydrogène ou un radical méthyle et R₁₄ représente un substituant choisi parmi l'hydrogène, un radical alkyle en C₁-C₆, un radical acétyle, un radical benzoyle, un radical phényle, un radical benzyle, un radical biphényle, un radical naphtyle, un radical tert-butyl-diméthylsilyle ou un radical tert-butyl-diphénylsilyle ; n représente un nombre entier de 0 à 1 ; et p représente un nombre entier de 3 à 50 ;
ou si R₁₃ représente un radical méthyle, R₁₄ représente également le groupement (-(CH₂-CH₂)_{q}-OR₁₅ où R₁₅ peut être choisi parmi l'hydrogène, un radical alkyle en C₁-C₆, un radical phényle, un radical benzyle ou un radical biphényle, où q représente un nombre entier de 1 à 20 ; et
R₃ est un substituant choisi parmi l'hydrogène, le brome, le chlore, le fluor, les groupes alkyle en C₁-C₆, cycloalkyle en C₃-C₇, thioalkyle en C₁-C₆, alcoxy en C₁-C₈, hydroxy, tert-butyl-diméthylsilyloxy, tert-butyl-diphénylsilyloxy, trifluorométhyle, phényle, phénylsilyloxy, tert-butyl-diphénylsilyloxy, tert-butylsilyloxy, tert-butylsilyloxy un radical tert-butyl-diméthylsilyloxy, un radical tert-butyl-diphénylsilyloxy, un radical trifluorométhyle, un radical phényle, un radical 4-méthoxyphényle, un radical phénoxy, un radical 4-méthoxyphénoxy, un radical benzyle, un radical 4-méthoxybenzyle, un radical benzyloxy, un radical 4-méthoxybenzyloxy, un radical biphényle, un radical biphényloxy, un radical naphtyle, un radical naphtoxy, un radical mono-alkyl(C₁-C₆)amino, un radical di-alkyl(C₁-C₆)amino un radical phénylamino, un radical (C₁-C₆)-alkyl-phénylamino, un radical diphénylamino, un radical (4-méthoxyphényl)amino, un radical ((C₁-C₆)-alkyl)-(4-méthoxyphényl)amino, un radical bis(4-méthoxyphényl)amino, un radical pipéridinyl un radical 3,5-diméthylpipéridinyle, un radical indolinyle, un radical morpholinyle, un radical 2,6-diméthylmorpholinyle, un radical thiomorpholinyle, un radical azacycloheptyle, un radical phénothiazinyle, un radical phénoxazinyle, un radical 1 ,2,3,4-tétrahydroquinolinyle, un radical 1 ,2,3,4-tétrahydroisoquinolinyle, un radical phénazinyle, un radical carbazolyle, un radical 1 ,2,3,4-tétrahydrocarbazolyle ou un radical 10,1-1-dihydro-dibenz[b,f]azépinyle, de préférence choisi parmi l'hydrogène, un radical alkyle en C₁-C₆, un radical cycloalkyle en C₃-C₇, un radical thioalkyle en C₁-C₆, un radical alcoxy en C₁-C₆, un radical hydroxy, un radical tert-butyl-diméthylsilyloxy, un radical tert-butyl-diphénylsilyloxy un radical trifluorométhyle, un radical phényle, un radical 4-méthoxyphényle, un radical phénoxy, un radical 4-méthoxyphénoxy, un radical benzyle, un radical 4-méthoxybenzyle, un radical benzyloxy ou un radical 4-méthoxybenzyloxy.

5. Utilisation d'un ou de plusieurs des naphtopyranes annelés photochromiques selon l'une des revendications précédentes 1 à 4 dans des matières plastiques de tous types, notamment à des fins ophtalmiques, dans des verres et des lunettes de tous types, de préférence des lunettes de vue, des lunettes pour automobilistes, des masques de ski, des lunettes de soleil, des masques de moto, pour des visières de casques de protection et similaires, ainsi qu'à des fins de protection solaire dans des véhicules et dans le secteur du bâtiment, sous la forme de fenêtres, d'écrans de protection, de couvertures, de toits.
